# EUROPEAN PATENT SPECIFICATION

(11) **EP 3 939 652 B1**
(45) Date of publication and mention of the grant of the patent: **11.06.2025**
(21) Application number: 19920118.7
(22) Date of filing: 15.03.2019
(51) Int. Cl.: A61N 1/05, A61N 1/39

(54) **INTRACARDIAC DEFIBRILLATION CATHETER**
INTRAKARDIALER DEFIBRILLATIONSKATHETER
CATHÉTER DE DÉFIBRILLATION INTRACARDIAQUE

(43) Date of publication of application: 19.01.2022
(73) Proprietor: Japan Lifeline Co., Ltd., Tokyo 140-0002 (JP)
(72) Inventor: SASAKI Takuya, Tokyo 140-0002 (JP)
(74) Representative: Grünecker Patent- und Rechtsanwälte PartG mbB
(86) International application number: PCT/JP2019/010909
(87) International publication number: WO 2020/188642

(56) References cited:
- WO-A1-2010/067719
- WO-A1-2011/102015
- WO-A1-2011/118061
- WO-A1-2018/083842
- JP-A- 2009 531 084
- JP-A- 2010 063 708
- JP-A- 2011 206 268
- JP-A- 2012 050 673
- JP-A- 2014 097 084
- JP-A- 2014 097 085
- JP-A- 2014 217 636
- JP-B2- 4 338 530
- US-A1- 2011 160 785
- US-A1- 2011 319 948

## Description

### Technical Field

The present invention relates to an intracardiac defibrillation catheter that is inserted into a cardiac cavity and that eliminates atrial fibrillation.

### Background Art

When atrial fibrillation occurs during cardiac catheterization surgery, electrical defibrillation needs to be performed.

The present applicant proposes, as a catheter for performing such defibrillation in a cardiac cavity, an intracardiac defibrillation catheter comprising an insulating tube member that has a multi-lumen structure, a handle that is connected to a proximal end of the tube member, a first DC electrode group that is made up of a plurality of ring-shaped electrodes mounted at a distal end portion of the tube member, a second DC electrode group that is made up of a plurality of ring-shaped electrodes spaced apart toward a proximal end side from the first DC electrode group and mounted at the distal end portion of the tube member, a first lead wire group that is made up of lead wires connected to each of the electrodes constituting the first DC electrode group, a second lead wire group that is made up of lead wires connected to each of the electrodes constituting the second DC electrode group, and an operating wire that, for flexing the distal end portion of the tube member and deflecting a distal end of the catheter, is eccentric with respect to a center axis of the tube member and extends in the tube member and has a rear end that can be operated by being pulled. The first lead wire group, the second lead wire group, and the operating wire respectively extend in different lumens of the tube member. When defibrillation is performed, voltages having polarities that differ from each other are applied to the first DC electrode group and the second DC electrode group (refer to Patent Literature 1 below).

By inserting a distal end portion of the intracardiac defibrillation catheter having such a structure from a superior vena cava into a right atrium, and by further inserting the distal end portion of the intracardiac defibrillation catheter into an opening of a coronary sinus (coronary sinus ostium) that exists at the lower back wall of the right atrium, the intracardiac defibrillation catheter is disposed so that the first DC electrode group is positioned in the coronary sinus and the second DC electrode group is positioned in the right atrium, after which voltages having polarities that differ from each other are applied to the first DC electrode group and the second DC electrode group. This makes it possible to apply electrical energy sufficient and required for defibrillation to a heart undergoing atrial fibrillation.

In addition, in recent years, the manipulation of inserting a distal end portion of an intracardiac defibrillation catheter from an inferior vena cava into a right atrium, forming a loop in the right atrium by the inserted distal end portion, and then inserting the distal end portion into a coronary sinus ostium has been performed. Such a manipulation (an approach from the inferior vena cava) has an invasiveness that is lower than the invasiveness of the manipulation of inserting an intracardiac defibrillation catheter from a superior vena cava into the right atrium and into the coronary sinus ostium (an approach from the superior vena cava), and is preferable from the viewpoint of postoperative appearance.

Conventionally, in order to realize good operability, intracardiac defibrillation catheters have been constituted so that the hardness of an insulating tube member increases stepwise from a distal end side toward a proximal end side (refer to, for example, paragraph [0027] of Patent Literature below). Further relevant prior art is described in US 2011/319948 A1, US 2011/160785 A1, WO 2010/067719 A1, WO 2011/102015 A1, WO 2011/118061 A1, JP 2014 097085 A, JP 2014 097084 A, WO 2018/083842 A1 and JP 2014 217636 A.

### Citation List

### Patent Literature

PTL 1: Japanese Unexamined Patent Application Publication No. 2010-63708

### Summary of Invention

### Technical Problem

However, it cannot be necessarily said that the operability in conventional intracardiac defibrillation catheters is good.

The present invention has been made based on such circumstances, and an object of the present invention is to provide an intracardiac defibrillation catheter that excels in operability, in particular, operability when a distal end portion of the catheter made by mounting a first electrode group is inserted into a coronary sinus.

### Solution to Problem

To this end, the present inventor has assiduously conducted studies repeatedly, and found out that, in a defibrillation catheter, since electrode groups (first electrode group and second electrode group) made by closely arranging ring-shaped electrodes having a wide width are mounted at a distal end portion of a tube member, the operability cannot be improved by merely increasing stepwise the hardness of the tube member from a distal end side toward a proximal end side, and good operability can be exhibited only after increasing stepwise from the distal end side toward the proximal end side the rigidity as a shaft at which electrodes (electrode groups) are mounted. Based on this finding, the present invention has been completed.

In particular it is provided an intracardiac defibrillation catheter having the features defined in claim 1. Further preferred embodiments are defined in the dependent claims. That is, an intracardiac defibrillation catheter of the present invention is a catheter that comprises an insulating tube member that is made up of a distal end side tube and a proximal end side tube, a handle that is connected to a proximal end of the tube member, a first DC electrode group that is made up of a plurality of ring-shaped electrodes mounted at the distal end side tube of the tube member, and a second DC electrode group that is made up of a plurality ring-shaped electrodes spaced apart toward a proximal end side from the first DC electrode group and mounted at the distal end side tube, the catheter performing defibrillation in a cardiac cavity by applying voltages having polarities that differ from each other to the first DC electrode group and the second DC electrode group, wherein, with regard to each of a first shaft portion to a fifth shaft portion below, when a three-point bending test is performed with a distance between fulcra being 70 mm and a flexing amount that results from applying a bending load to an intermediate point of the distance being 10 mm, bending loads L1 to L5 measured for each of the shaft portions have a relationship of L1 < L2 < L3 < L4 < L5.

- First shaft portion: a shaft portion that extends from a distal end of a catheter shaft to a proximal end position of the first DC electrode group
- Second shaft portion: a shaft portion that extends from the proximal end position of the first DC electrode group to a distal end position of the second DC electrode group
- Third shaft portion: a shaft portion that extends from the distal end position of the second DC electrode group to a proximal end position of the second DC electrode group
- Fourth shaft portion: a shaft portion that extends from the proximal end position of the second DC electrode group to a proximal end position of the distal end side tube
- Fifth shaft portion: a shaft portion made from the proximal end side tube

According to the intracardiac defibrillation catheter having such a structure, since the bending rigidity as a shaft at which the electrodes (electrode groups) are mounted increases stepwise from a distal end side toward the proximal end side, it is possible to exhibit excellent operability.

In the intracardiac defibrillation catheter of the present invention, it is preferable that a bending load L1 of the first shaft portion be 120 gf or less, and a bending load L4 of the fourth shaft portion be 350 gf or greater (1 gf = 0.00980665 N).

The first shaft portion whose bending load L1 is 120 gf or less has bendability and, in particular, excels in insertability into a coronary sinus.

In addition, when the bending load L4 of the fourth shaft portion is 350 gf or greater, it is possible to clearly set a change (an increase) in the bending rigidity from the first shaft portion to the fourth shaft portion.

In the intracardiac defibrillation catheter of the present invention, it is preferable that, when a surface hardness of the distal end side tube that constitutes the first shaft portion is H1 and a surface hardness of the distal end side tube that constitutes the second shaft portion is H2, a difference between H2 and H1 be 16D to 35D, in particular, 20D to 30D.

When the hardness difference between H2 and H1 is 16D or greater, the bending rigidity in the second shaft portion at which an electrode group is not mounted can be reliably made larger than the bending rigidity in the first shaft portion that is made by mounting the first DC electrode group.

In addition, when the hardness difference is within 35D, it is possible to prevent the bending rigidity in the second shaft portion from becoming too large.

As a result, it is possible to further improve the operability, in particular, the operability when the distal end portion that is made by mounting the first DC electrode group is inserted into a coronary sinus.

Further, it is preferable that, when a surface hardness of the distal end side tube that constitutes the third shaft portion is H3 and a surface hardness of the distal end side tube that constitutes the fourth shaft portion is H4, a difference between H4 and H3 is 2D to 16D, in particular, 5D to 10D.

When the hardness difference between H4 and H3 is 2D or greater, the bending rigidity in the fourth shaft portion at which an electrode group is not mounted can be reliably made larger than the bending rigidity in the third shaft portion that is made by mounting the second DC electrode group.

In addition, when the hardness difference is within 16D, it is possible to prevent the bending rigidity in the fourth shaft portion from becoming too large.

As a result, it is possible to further improve the operability.

### Advantageous Effects of Invention

The intracardiac defibrillation catheter of the present invention excels in operability, in particular, operability when a tube portion at which the first DC electrode group is mounted is inserted into a coronary sinus.

### Brief Description of Drawings

[Fig. 1] Fig. 1 is a plan view showing a defibrillation catheter according to an embodiment of the present invention.
[Fig. 2] Fig. 2 is a cross sectional view (sectional view along II-II in Fig. 1) of a tube member that constitutes the defibrillation catheter shown in Fig. 1.
[Fig. 3] Fig. 3 is a cross sectional view (sectional view along III-III in Fig. 1) of the tube member that constitutes the defibrillation catheter shown in Fig. 1.
[Fig. 4] Fig. 4 is a graph showing test results of a three-point bending test performed on defibrillation catheters of the present invention and on conventional publicly known defibrillation catheters.

### Description of Embodiments

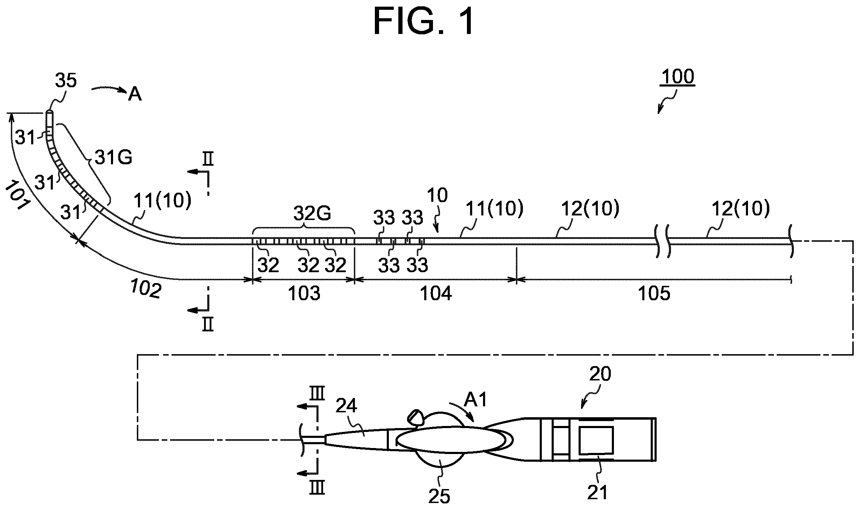

A defibrillation catheter 100 of this embodiment shown in Figs. 1 to 3 is a catheter that comprises an insulating tube member 10 that is made up of a distal end side tube 11 and a proximal end side tube 12, a control handle 20 that is connected to a proximal end of the tube member 10, a distal end tip 35 that is fixed to a distal end of the tube member 10, a first DC electrode group 31G that is made up of eight ring-shaped electrodes 31 mounted at the distal end side tube 11 of the tube member 10, a second DC electrode group 32G that is made up of eight ring-shaped electrodes 32 spaced apart toward a proximal end side from the first DC electrode group 31G and mounted at the distal end side tube 11, four ring-shaped electrodes 33 for measuring electrical potential that are mounted at the distal end side tube 11 on a proximal end side of the second DC electrode group 32G, a first lead wire group 41G that is made up of eight lead wires 41 connected to each of the electrodes 31 constituting the first DC electrode group 31G, a second lead wire group 42G that is made up of eight lead wires 42 connected to each of the electrodes 32 constituting the second DC electrode group 32G, four lead wires 43 that are connected to each of the ring-shaped electrodes 33 for measuring electrical potential, and an operating wire 70 that, for flexing a distal end portion of the tube member 10, is eccentric with respect to a center axis of the tube member 10 and extends in the tube member 10, has a distal end that is connected and fixed to the distal end tip 35, and has a rear end that can be operated by being pulled. The catheter performs defibrillation in a cardiac cavity by applying voltages having polarities that differ from each other between the first DC electrode group 31G and the second DC electrode group 32G,
wherein with regard to each of a first shaft portion 101 that extends from a distal end of a catheter shaft to a proximal end position of the first DC electrode group 31G, a second shaft portion 102 that extends from the proximal end position of the first DC electrode group 31G to a distal end position of the second DC electrode group 32G, a third shaft portion 103 that extends from the distal end position of the second DC electrode group 32G to a proximal end position of the second DC electrode group 32G, a fourth shaft portion 104 that extends from the proximal end position of the second DC electrode group 32G to a proximal end position of the distal end side tube 11, and a fifth shaft portion 105 that is made from the proximal end side tube 12, when a three-point bending test is performed with the distance between fulcra being 70 mm and the flexing amount that results from applying a bending load to an intermediate point of the distance being 10 mm, bending loads L1, L2, L3, L4, and L5 measured for the first shaft portion 101, the second shaft portion 102, the third shaft portion 103, the fourth shaft portion 104, and the fifth shaft portion 105, respectively, have the relationship of L1 < L2 < L3 < L4 < L5.

The defibrillation catheter 100 of the embodiment comprises the tube member 10, the control handle 20, the distal end tip 35, the first DC electrode group 31G, the second DC electrode group 32G, the electrodes 33 for measuring electrical potential, the first lead wire group 41G, the second lead wire group 42G, the lead wires 43, and the operating wire 70.

The tube member 10 that constitutes the defibrillation catheter 100 is made up of the distal end side tube 11 and the proximal end side tube 12, and is an insulating tube member that has a multi-lumen structure.

The outside diameter of the tube member 10 (the distal end side tube 11 and the proximal end side tube 12) is, for example, 1.7 to 2.4 mm, with a preferred example being 2.0 mm.

The effective length of the tube member 10 is, for example, 600 to 1100 mm, with a preferred example being 650 mm.

The length of the distal end side tube 11 is, for example, 150 to 300 mm, with a preferred example being 239 mm.

The length of the proximal end side tube 12 is, for example, 300 to 950 mm, with a preferred example being 411 mm.

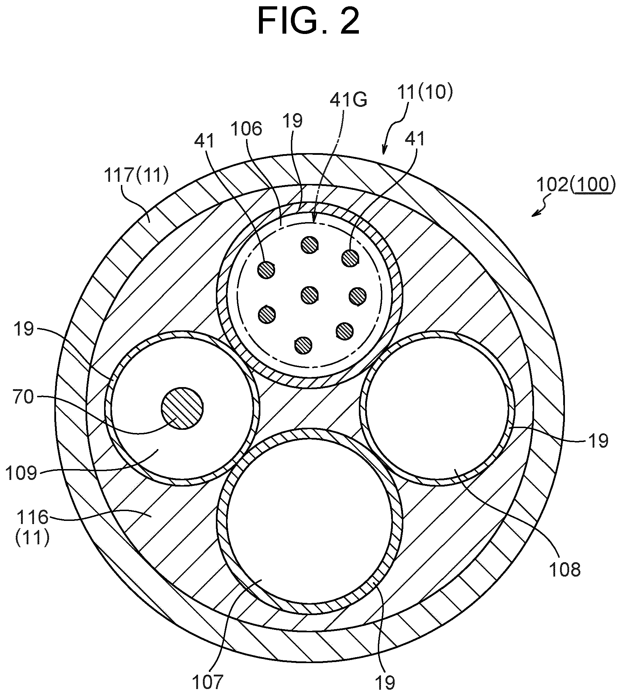

As shown in Fig. 2, the distal end side tube 11 that constitutes the tube member 10 comprises an inner part 116 and an outer part 117 that covers the inner part 116.

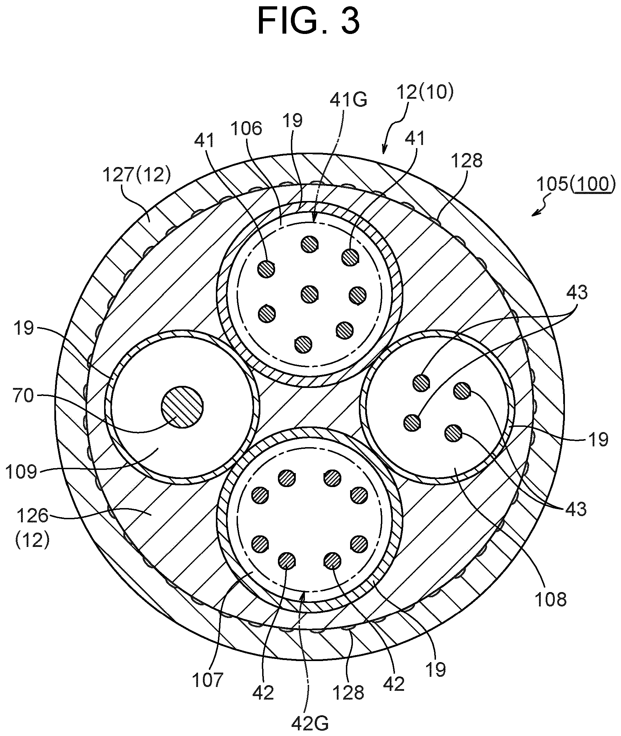

As shown in Fig. 3, the proximal end side tube 12 that constitutes the tube member 10 comprises an inner part 126, an outer part 127 that covers the inner part 126, and a braid 128 that is embedded in the outer part 127.

Resin that constitutes each of the inner part 116 and the outer part 117 of the distal end side tube 11 and resin that constitutes each of the inner part 126 and the outer part 127 of the proximal end side tube 12 can be a thermoplastic polyamide-based elastomer, such as polyether block amide (PEBAX) or nylon.

The braid 128 that constitutes the proximal end side tube 12 can be a metal material, such as stainless steel, or a resin material, such as PEEK.

It is preferable that the hardness of the resin that constitutes the inner part 116 of the distal end side tube 11 and the hardness of the resin that constitutes the inner part 126 of the proximal end side tube 12 be 25D to 74D, with a preferred example being 63D. The hardness of the inner part 116 and the hardness of the inner part 126 may be the same as or may differ from each other.

As shown in Figs. 2 and 3, four lumens 106 to 109 are each formed by being partitioned by a lumen tube 19 made of fluorine-based resin at the tube member 10 (the distal end side tube 11 and the proximal end side tube 12).

The fluorine-based resin that constitutes the lumen tube 19 can be perfluoroalkyl vinyl ether copolymer (PFA) or polytetrafluoroethylene (PTFE).

The hardness of the resin that constitutes the outer part 117 of the distal end side tube 11 and the hardness of the resin that constitutes the outer part 127 of the proximal end side tube 12 (the surface hardness of the tube member 10) increase stepwise from a distal end side toward the proximal end side.

Here, an example of a change in the hardness of the resin that constitutes the outer part 117 and a change in the hardness of the resin that constitutes the outer part 127 is one in which, when the shaft of the defibrillation catheter 100 is divided into the first shaft portion 101 that extends from the distal end of the catheter shaft to the proximal end position of the first DC electrode group 31G (the electrode 31 at a proximal-most end), the second shaft portion 102 that extends from the proximal end position of the first DC electrode group 31G to the distal end position of the second DC electrode group 32G (the electrode 32 at a distal-most end), the third shaft portion 103 that extends from the distal end position of the second DC electrode group 32G to the proximal end position of the second DC electrode group 32G (the electrode 32 at a proximal-most end), the fourth shaft portion 104 that extends from the proximal end position of the second DC electrode group 32G to the proximal end position of the distal end side tube 11 (a distal end position of the proximal end side tube 12), and the fifth shaft portion 105 that is made from the proximal end side tube 12 (and that extends from the distal end position of the proximal end side tube 12 to a strain relief 24), the hardness (H1) of the resin that constitutes the outer part 117 in the first shaft portion 101 is 40D, the hardness (H2) of the resin that constitutes the outer part 117 in the second shaft portion 102 is 63D, the hardness (H3) of the resin that constitutes the outer part 117 in the third shaft portion 103 is 63D, the hardness (H4) of the resin that constitutes the outer part 117 in the fourth shaft portion 104 is 72D, and the hardness (H5) of the resin that constitutes the outer part 127 in the fifth shaft portion 105 is 74D.

Note that a change in the hardness of the resin that constitutes the outer part 117 and the hardness of the resin that constitutes the outer part 127 is not limited to the example above as long as the relationship of H1 < H2 ≤ H3 < H4 < H5 is established and the relationship of L1 < L2 < L3 < L4 < L5 described below can be established.

In addition, the hardness of a boundary region between shaft portions that are adjacent to each other may change with a gradient.

The difference between the hardness (H2) and the hardness (H1) is preferably 16D to 35D and particularly preferably 20D to 30D.

When the hardness difference is 16D or greater, the bending rigidity of the second shaft portion 102 at which an electrode group is not mounted can be reliably made greater than the bending rigidity of the first shaft portion 101 that is made by mounting the first DC electrode group 31G.

In addition, when the hardness difference is within 35D, it is possible to prevent the bending rigidity of the second shaft portion 102 from becoming too large.

The difference between the hardness (H4) and the hardness (H3) is preferably 2D to 16D and particularly preferably 5D to 10D.

When the difference is 2D or greater, the bending rigidity of the fourth shaft portion 104 at which an electrode group is not mounted can be reliably made greater than the bending rigidity of the third shaft portion 103 at which the second DC electrode group 32G is mounted.

In addition, when the hardness difference is within 16D, it is possible to prevent the bending rigidity of the fourth shaft portion 104 from becoming too large.

The length of the first shaft portion 101 of the defibrillation catheter 100 is, for example, 40 to 70 mm, with a preferred example being 52 mm.

The length of the second shaft portion 102 is, for example, 40 to 100 mm, with a preferred example being 72 mm.

The length of the third shaft portion 103 is, for example, 40 to 72 mm, with a preferred example being 50 mm.

The length of the fourth shaft portion 104 is, for example, 50 to 80 mm, with a preferred example being 65 mm.

The length of the fifth shaft portion 105 is, for example, 300 to 950 mm, with a preferred example being 411 mm.

As shown in Fig. 1, the control handle 20 that constitutes the defibrillation catheter 100 has a handle body 21, a rotation operating part 25, and the strain relief 24.

By rotating the rotation operating part 25 in the direction of arrow A1 in Fig. 1, it is possible to pull the rear end of the operating wire 70 described below.

The first DC electrode group 31G is mounted at the distal end side tube 11 (a structural portion of the first shaft portion 101) that constitutes the tube member 10.

In addition, the second DC electrode group 32G is mounted at the distal end side tube 11 (a structural portion of the third shaft portion 103).

In the present invention, "electrode group" refers to an assembly of a plurality of electrodes that are mounted at narrow intervals (for example, 5 mm or less) with the same pole being constituted (with the same polarity), or for the same purpose.

The first DC electrode group is made by mounting, in a distal end portion of the tube member, a plurality of electrodes that constitute the same pole (negative pole or positive pole) at narrow intervals. Here, the number of electrodes that constitute the first DC electrode group, though differing depending upon the width of the electrodes and the arrangement interval of the electrodes, is, for example, 4 to 13, and is preferably 8 to 10.

In the present embodiment, the first DC electrode group 31G is constituted by eight ring-shaped electrodes 31. The electrodes 31 that constitute the first DC electrode group 31G are connected to terminals of the same pole in a direct-current power supply device through the lead wires (the lead wires 41 that constitute the first lead wire group 41G shown in Figs. 2 and 3) and a connector that is built in a proximal end portion of the control handle 20.

Here, it is preferable that the width (the length in an axial direction) of electrode 31 be 2 to 5 mm, with a preferred example being 4 **mm.**

When the width of electrode 31 is too narrow, the heat generation amount when a voltage is applied becomes too large, and surrounding tissue may be damaged. On the other hand, when the width of electrode 31 is too wide, a portion in the tube member 10 at which the first DC electrode group 31G is mounted may lose its flexibility/bendability, or the sensitivity with which electrical potential information is detected may be reduced when the electrodes 31 are used for measuring electrical potential as described below.

It is preferable that the mounting interval between the electrodes 31 (the separation distance between the electrodes that are adjacent to each other) be 1 to 5 mm, with a preferred example being 2 mm.

When using the intracardiac defibrillation catheter 100 (when the intracardiac defibrillation catheter 100 is disposed in a cardiac cavity), the first DC electrode group 31G is positioned in a coronary sinus (CS).

The second DC electrode group is made by mounting at narrow intervals, in the distal end portion of the tube member spaced apart toward the proximal end side from the mounting position of the first DC electrode group, a plurality of electrodes that constitute a pole (positive pole or negative pole) opposite to that of the first DC electrode group. Here, the number of electrodes that constitute the second DC electrode group, though differing depending upon the width of the electrodes and the arrangement interval of the electrodes, is, for example, 4 to 13, and is preferably 8 to 10.

In the present embodiment, the second DC electrode group 32G is constituted by eight ring-shaped electrodes 32. The electrodes 32 that constitute the second DC electrode group 32G are connected to terminals having the same pole (terminals having a pole that is opposite to that of the terminals to which the first DC electrode group 31G is connected) in a direct-current power supply device through the lead wires (the lead wires 42 that constitute the second lead wire group 42G shown in Fig. 3) and a connector that is built in the proximal end portion of the control handle 20.

Therefore, voltages having polarities that differ from each other are applied to the first DC electrode group 31G (the electrodes 31) and the second DC electrode group 32G (the electrodes 32), and the first DC electrode group 31G and the second DC electrode group 32G become electrode groups having polarities that differ from each other (when one of the electrode groups has a negative pole, the other electrode group has a positive pole).

Here, it is preferable that the width (the length in an axial direction) of electrode 32 be 2 to 5 mm, with a preferred example being 4 mm.

When the width of electrode 32 is too narrow, the heat generation amount when a voltage is applied becomes too large, and surrounding tissue may be damaged. On the other hand, when the width of electrode 32 is too wide, a portion of the tube member 10 at which the second DC electrode group 32G is mounted may lose its flexibility/bendability, or the sensitivity with which electrical potential information is detected may be reduced when the electrodes 32 are used for measuring electrical potential as described below.

It is preferable that the mounting interval between the electrodes 32 (the separation distance between the electrodes that are adjacent to each other) be 1 to 5 mm, with a preferred example being 2 mm.

When using the intracardiac defibrillation catheter 100 (when the intracardiac defibrillation catheter 100 is disposed in a cardiac cavity), the second DC electrode group 32G is positioned in a right atrium (RA).

Note that the electrodes that constitute the first DC electrode group 31G and the electrodes that constitute the second DC electrode group can be used for measuring electrical potential.

Four electrodes 33 are mounted for measuring electrical potential at the distal end side tube 11 (a structural portion of the fourth shaft portion 104) on the proximal end side of the second DC electrode group 32G.

The electrodes 33 that are mounted on the proximal end side of the second DC electrode group 32G are connected to an electrocardiograph through the lead wires (the lead wires 43 shown in Fig. 3) and a connector that is built in the proximal end portion of the control handle 20.

Here, it is preferable that the width (the length in an axial direction) of electrode 33 be 0.5 to 2.0 mm, with a preferred example being 1.2 mm.

When the width of electrode 33 is too wide, the measurement accuracy of a cardiac potential is reduced, and it becomes difficult to identify the site of occurrence of an abnormal potential.

The distal end tip 35 is mounted at the distal end of the tube member 10.

A lead wire is not connected to the distal end tip 35, and, in the present embodiment, the distal end tip 35 is not used as an electrode. However, by connecting a lead wire, the distal end tip 35 can also be used as an electrode. The constituent material of the distal end tip 35 is, for example, a metal material, such as platinum or stainless steel, or various resin materials, and is not particularly limited.

In order to realize good contrast property with respect to X rays, it is preferable that the electrodes 31 that constitute the first DC electrode group 31G, the electrodes 32 that constitute the second DC electrode group 32G, and the electrodes 33 for measuring electrical potential be made of platinum or a platinum-based alloy.

The first lead wire group 41G shown in Figs. 2 and 3 is an assembly of eight lead wires 41 that are connected to each of the eight electrodes 31 that constitute the first DC electrode group 31G.

By the first lead wire group 41G (the lead wires 41), each of the eight electrodes 31 that constitute the first DC electrode group 31G can be electrically connected to the direct-current power supply device.

The eight electrodes 31 that constitute the first DC electrode group 31G are connected to the respective different lead wires 41. Each of the lead wires 41 is, at a distal end thereof, welded to an inner peripheral surface of electrode 31, and enters a first lumen 106 from a side hole that is formed in a tubular wall of the tube member 10. The eight lead wires 41 that have entered the first lumen 106 extend to the first lumen 106 as the first lead wire group 41G and enter the inside of the control handle 20.

The second lead wire group 42G shown in Fig. 3 is an assembly of eight lead wires 42 that are connected to each of the eight electrodes 32 that constitute the second DC electrode group 32G.

By the second lead wire group 42G (the lead wires 42), each of the eight electrodes 32 that constitute the second DC electrode group 32G can be electrically connected to the direct-current power supply device.

The eight electrodes 32 that constitute the second DC electrode group 32G are connected to the respective different lead wires 42. Each of the lead wires 42 is, at a distal end thereof, welded to an inner peripheral surface of electrode 32, and enters a second lumen 107 from a side hole that is formed in a tubular wall of the tube member 10. The eight lead wires 42 that have entered the second lumen 107 extend to the second lumen 107 as the second lead wire group 42G and enter the inside of the control handle 20.

As described above, the first lead wire group 41G (the eight lead wires 41) extends in the first lumen 106 and the second lead wire group 42G (the eight lead wires 42) extends in the second lumen 107, whereby the first lead wire group 41G and the second lead wire group 42G can be insulated and isolated from each other in the tube member. Therefore, when a voltage required for intracardiac defibrillation is applied, it is possible to reliably prevent occurrence of a short circuit between the first lead wire group 41G (the first DC electrode group 31G) and the second lead wire group 42G (the second DC electrode group 32G).

The four lead wires 43 shown in Fig. 3 are, respectively, connected to the four electrodes 33 for measuring electrical potential.

The four lead wires 43 are, at respective distal ends thereof, welded to an inner peripheral surface of electrode 33, and enter a third lumen 108 from a side hole that is formed in a tubular wall of the tube member 10, extend to the third lumen 108, and enter the inside of the control handle 20. By the lead wires 43, each of the electrodes 33 can be connected to an electrocardiograph.

The lead wires 41, the lead wires 42, and the lead wires 43 are all made up of a resin coated wire in which an outer peripheral surface of a metal conductive wire is coated with resin, such as polyimide. Here, the film thickness of the coating resin is approximately 2 to 30 µm.

The defibrillation catheter 100 of the present embodiment comprises the operating wire 70 for flexing the distal end portion of the tube member 10.

Although the operating wire 70 is constituted by stainless steel or a Ni-Ti-based super-elastic alloy, the operating wire 70 does not necessarily need to be constituted by a metal, and may be constituted by, for example, a high-strength non-conductive wire.

As shown in Figs. 2 and 3, the operating wire 70 is inserted so as to be movable in a tube axis direction in the fourth lumen 109 of the tube member 10.

A distal end of the operating wire 70 is connected and fixed to the distal end tip 35 with solder filled in an internal space of the distal end tip 35.

A rear end of the operating wire 70 is connected and fixed to the rotation operating part 25 of the control handle 20 and can be operated by being pulled.

By rotating the rotation operating part 25 in the direction of arrow A1 in Fig. 1, the rear end of the operating wire 70 is pulled to make it possible to flex the distal end portion of the tube member 10 in the direction of arrow A in Fig. 1.

The defibrillation catheter 100 of the present embodiment is constituted so that the bending rigidity of the shaft is increased stepwise from the distal end side toward the proximal end side.

Specifically, with regard to each of the first shaft portion 101, the second shaft portion 102, the third shaft portion 103, the fourth shaft portion 104, and the fifth shaft portion 105 of the defibrillation catheter 100, when a three-point bending test is performed with the distance between fulcra being 70 mm and the flexing amount that results from applying a bending load to an intermediate point of the distance being 10 mm, the bending loads L1 to L5 measured for each of the shaft portions have the relationship of L1 < L2 < L3 < L4 < L5.

In this way, by increasing the surface hardness of the tube member 10 (the hardness of the resin that constitutes the outer part 117 of the distal end side tube 11 and the hardness of the resin that constitutes the outer part 127 of the proximal end side tube 12) stepwise from the distal end side toward the proximal end side and by causing the bending rigidity of the shaft at which the electrodes (the electrode groups) are mounted to increase stepwise from the distal end side toward the proximal end side, it is possible to remarkably improve the operability compared with the operability of conventional defibrillation catheters.

In the defibrillation catheter 100, it is preferable that the value of the bending load L1 of the first shaft portion 101 that results from the three-point bending test above be 120 gf or less.

The first shaft portion 101 whose bending load L1 is 120 gf or less has good flexibility/bendability, and, in particular, excels in insertability into a coronary sinus.

In addition, it is preferable that the bending load L4 of the fourth shaft portion 104 that results from the three-point bending test above be 350 gf or greater.

When the value of the bending load L4 of the fourth shaft portion 104 is 350 gf or greater, it is possible to clearly set a change (an increase) in the bending rigidity from the first shaft portion 101 to the fourth shaft portion 104.

Although one embodiment of the present invention has been described above, the defibrillation catheter of the present invention is not limited to such embodiments and can be variously changed.

For example, the defibrillation catheter of the present invention may be a bi-directional type that comprises two operating wires.

In addition, the tube member that constitutes the defibrillation catheter of the present invention may be a single lumen structure.

### EXAMPLES

Defibrillation catheters 100 of the present embodiment having a form such as that shown in Figs. 1 to 3 and the specifications given below were manufactured.

### (Specifications)

- Effective length of catheter shaft (tube member 10): 650 mm
- Length of shaft portions that are constituted by distal end side tube 11: 239 mm
- Length of shaft portion that is constituted by proximal end side tube 12: 411 mm
- Material of inner parts 116 and 126: PEBAX (hardness: 63D)
- Material of outer parts 117 and 127: PEBAX (hardness: as mentioned below)
- Material of braid 128: stainless steel

- Hardness (H1) of resin that constitutes outer part 117 of first shaft portion 101: 40D
- Hardness (H2) of resin that constitutes outer part 117 of second shaft portion 102: 63D
- Hardness (H3) of resin that constitutes outer part 117 of third shaft portion 103: 63D
- Hardness (H4) of resin that constitutes outer part 117 of fourth shaft portion 104: 72D
- Hardness (H5) of resin that constitutes outer part 127 of fifth shaft portion 105: 74D
- Hardness difference (H2-H1): 23D
- Hardness difference (H4-H3): 9D

- Length of first shaft portion 101: 52 mm
- Length of second shaft portion 102: 72 mm
- Length of third shaft portion 103: 50 mm
- Length of fourth shaft portion 104: 65 mm
- Length of fifth shaft portion 104: 411 mm

- Width of electrode 31 that constitutes first DC electrode group 31G: 4 mm
- Mounting interval between electrodes 31: 2 mm
- Width of electrode 32 that constitutes second DC electrode group 32G: 4 mm
- Mounting interval between electrodes 32: 2 mm
- Width of electrode 33: 1.2 mm

Two defibrillation catheters 100 having the structure above were prepared (Examples 1 and 2), and a three-point bending test was performed on each of the defibrillation catheters 100 to measure the bending loads L1 to L5 of the respective first shaft portion 101 to fifth shaft portion 105.

As the three-point bending test, two supports were disposed with a separation distance between the two supports (distance between fulcra) being 70 mm, and a shaft was placed on the supports so that each shaft portion whose bending load was to be measured was positioned at an intermediate point between the two supports. Next, a bending load in a direction perpendicular to an axial direction of the shaft was applied to a position on the shaft, placed on the supports, corresponding to the intermediate point between the supports, to determine the relationship between the flexing amount, being a displacement in the perpendicular direction, and the magnitude of the bending load measured by a load meter and to measure the bending load of the shaft (each shaft portion to be measured) when the flexing amount became 10 mm. Here, the test was performed at room temperature and the flexing speed was 50 m/min.

Note that, regarding the first shaft portion 101 and the third shaft portion 103, a bending load was applied to each of the first shaft portion 101 and the third shaft portion 103 from the ring-shaped electrodes that constitute the electrode groups.

Note that, in the "three-point bending test" prescribed in the present invention, as long as a shaft portion whose bending load is to be measured is positioned at the intermediate point between the two supports, shaft portions that are adjacent to the shaft portion to be measured may be positioned on the two supports.

On the other hand, three commercially available defibrillation catheters were prepared (Comparative Examples 1 to 3), and bending loads L1 to L5 were measured in the same way as described above.

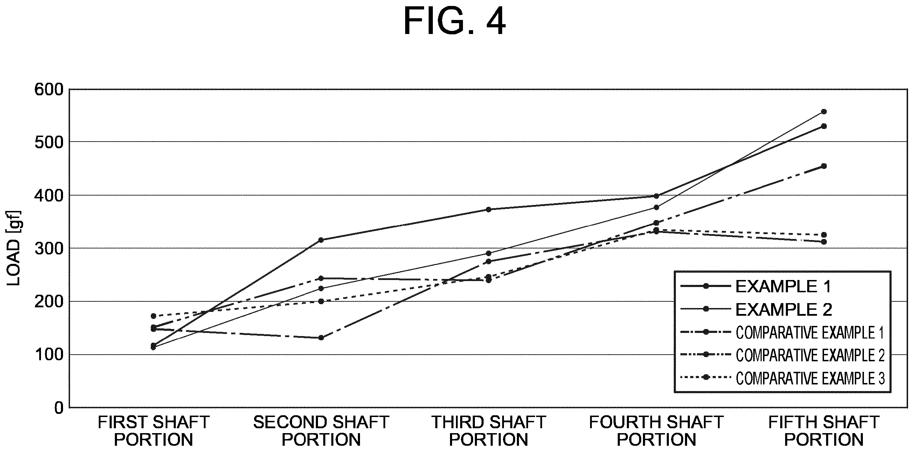

The results are shown in Table 1 below and Fig. 4.

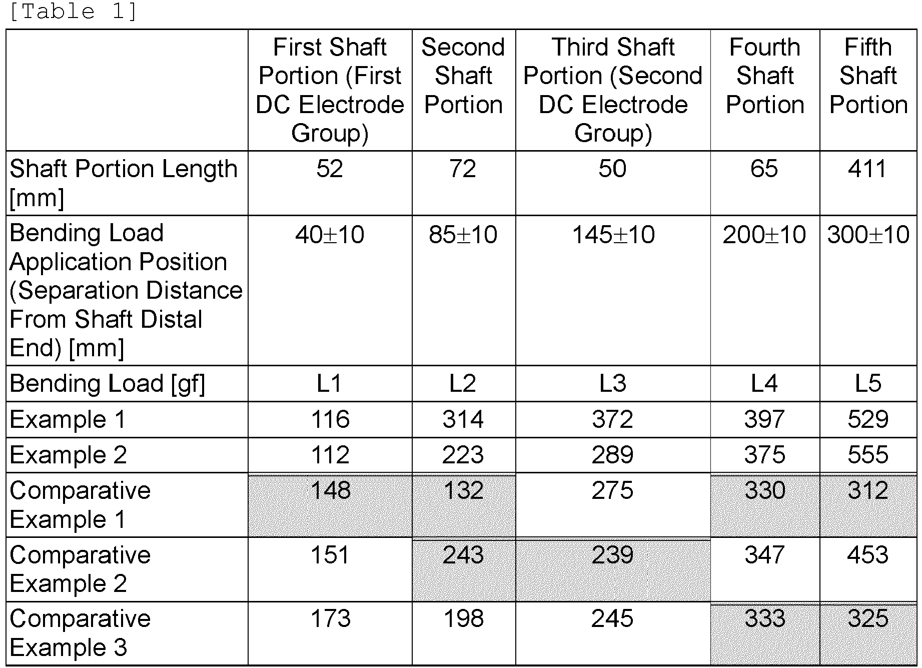

As shown in Table 1 and Fig. 4, the defibrillation catheters 100 of Examples 1 and 2 are such that the shaft bending rigidity increases stepwise from the distal end side toward the proximal end side (the relationship of L1 < L2 < L3 < L4 < L5 is established).

In addition, the defibrillation catheters 100 of Examples 1 and 2 excelled in operability when the first DC electrode group 31G was disposed in a coronary sinus and the second DC electrode group 32G was disposed in a right atrium.

In contrast, in the defibrillation catheter of Comparative Example 1, the bending rigidity in the second shaft portion was lower than the bending rigidity of the first shaft portion and the bending rigidity in the fifth shaft portion was lower than the bending rigidity of the fourth shaft portion; in the defibrillation catheter of Comparative Example 2, the bending rigidity in the third shaft portion was lower than the bending rigidity of the second shaft portion; and, in the defibrillation catheter of Comparative Example 3, the bending rigidity in the fifth shaft portion was lower than the bending rigidity of the fourth shaft portion.

The defibrillation catheters of Comparative Examples 1 to 3 were inferior in operability due to a kink occurring easily between shaft portions whose relationship between the bending rigidities were reversed.

### Reference Signs List

100 defibrillation catheter
10 tube member
101 first shaft portion
102 second shaft portion
103 third shaft portion
104 fourth shaft portion
105 fifth shaft portion
106 first lumen
107 second lumen
108 third lumen
109 fourth lumen
11 distal end side tube
116 inner part of distal end side tube
117 outer part of distal end side tube
12 proximal end side tube
126 inner part of proximal end side tube
127 outer part of proximal end side tube
128 braid
20 control handle
25 rotation operating part
35 distal end tip
31G first DC electrode group
31 ring-shaped electrode constituting first DC electrode group
32G second DC electrode group
32 ring-shaped electrode constituting second DC electrode group
33 ring-shaped electrode
41G first lead wire group
41 lead wire constituting first lead wire group
42G second lead wire group
42 lead wire constituting second lead wire group
43 lead wire constituting third lead wire group
70 operating wire

## Claims

1. An intracardiac defibrillation catheter (100) having a shaft and comprising an insulating tube member (10) that is made up of a distal end side tube (11) and a proximal end side tube (12), a handle (20) that is connected to a proximal end of the tube member (10), a first electrode group (31G) that is made up of a plurality of ring-shaped electrodes (31) mounted at the distal end side tube (11) of the tube member (10), and a second electrode group (32G) that is made up of a plurality of ring-shaped electrodes (32) spaced apart toward a proximal end side from the first electrode group (31G) and mounted at the distal end side tube (11),
the catheter (100) being configured to perform defibrillation in a cardiac cavity by applying voltages having polarities that differ from each other to the first electrode group (31G) and the second electrode group (32G), wherein, with regard to each of a first shaft portion (101), a second shaft portion (102), a third shaft portion (103), a fourth shaft portion (104), and a fifth shaft portion (105), when a three-point bending test is performed with a distance between fulcra being 70 mm and a flexing amount that results from applying a bending load to an intermediate point of the distance being 10 mm, bending loads L1 to L5 measured for each of the first to fifth shaft portions (101, 102, 103, 104, 105) have a relationship of L1 < L2 < L3 < L4 < L5, wherein
- the first shaft portion (101) is a shaft portion that extends from a distal end of a catheter shaft of the intracardiac defibrillation catheter (100) to a proximal end position of the first electrode group (31G),
- the second shaft portion (102) is a shaft portion that extends from the proximal end position of the first electrode group (31G) to a distal end position of the second electrode group (32G),
- the third shaft portion (103) is a shaft portion that extends from the distal end position of the second electrode group (32G) to a proximal end position of the second electrode group (32G),
- the fourth shaft portion (104) is a shaft portion that extends from the proximal end position of the second electrode group (32G) to a proximal end position of the distal end side tube (11),
- and the fifth shaft portion (105) is a shaft portion that extends along the proximal end side tube (12).

2. The intracardiac defibrillation catheter (100) according to Claim 1, wherein the bending load L1 of the first shaft portion (101) is 120 gf or less, and wherein the bending load L4 of the fourth shaft portion (104) is 350 gf or greater (1 gf = 0.00980665 N).

3. The intracardiac defibrillation catheter (100) according to Claim 1 or Claim 2, wherein, when a surface hardness of the distal end side tube (11) that constitutes the first shaft portion (101) is H1 and a surface hardness of the distal end side tube that constitutes the second shaft portion (102) is H2, a difference between H2 and H1 is 16D to 35D.

4. The intracardiac defibrillation catheter (100) according to Claim 3, wherein, when a surface hardness of the distal end side tube that constitutes the third shaft portion (103) is H3 and a surface hardness of the distal end side tube that constitutes the fourth shaft portion (104) is H4, a difference between H4 and H3 is 2D to 16D.

5. The intracardiac defibrillation catheter (100) according to any one of Claims 1 to 4, wherein when the intracardiac defibrillation catheter (100) is disposed in the cardiac cavity the first electrode group (31G) is positioned in a coronary sinus and the second electrode group (32G) is positioned in a right atrium.

## Patentansprüche

1. Intrakardialer Defibrillationskatheter (100), der einen Schaft aufweist und umfassend ein isolierendes Rohrelement (10),
das aus einem distalen Endseitenrohr (11) und einem proximalen Endseitenrohr (12) hergestellt ist, einen Griff (20), der mit einem proximalen Ende des Rohrelements (10) verbunden ist, eine erste Elektrodengruppe (31G), die aus einer Vielzahl von ringförmigen Elektroden (31) hergestellt ist, die an dem distalen Endseitenrohr (11) des Rohrelements (10) montiert sind, und eine zweite Elektrodengruppe (32G), die aus einer Vielzahl von ringförmigen Elektroden (32) hergestellt ist, die von der ersten Elektrodengruppe (31G) zu einer proximalen Endseite hin beabstandet sind und an dem distalen Endseitenrohr (11) montiert sind,
wobei der Katheter (100) konfiguriert ist, um eine Defibrillation in einer Herzhöhle durch Anlegen von Spannungen, die Polaritäten aufweisen, die sich voneinander unterscheiden, an die erste Elektrodengruppe (31G) und die zweite Elektrodengruppe (32G) durchzuführen, wobei in Bezug auf jeweils einen ersten Schaftabschnitt (101), einen
zweiten Wellenabschnitt (102), einen dritten Wellenabschnitt (103), einen vierten Wellenabschnitt (104) und einen fünften Wellenabschnitt (105)
wenn ein Dreipunktbiegeversuch mit einem Abstand zwischen den Drehpunkten, der 70 mm beträgt, und einem Biegebetrag, der sich aus dem Anwenden einer Biegelast auf einen Zwischenpunkt des Abstands ergibt, der 10 mm beträgt, durchgeführt wird, Biegelasten L1 bis L5, die für jeden des ersten bis fünften Schaftabschnitts (101, 102, 103, 104, 105) gemessen wird, eine Beziehung von L1 < L2 < L3 < L4 < L5 aufweisen, wobei
- der erste Schaftabschnitt (101) ein Schaftabschnitt ist, der sich von einem distalen Ende eines Katheterschafts des intrakardialen Defibrillationskatheters (100) zu einer proximalen Endposition der ersten Elektrodengruppe (31G) erstreckt,
- der zweite Schaftabschnitt (102) ein Schaftabschnitt ist, der sich von der proximalen Endposition der ersten Elektrodengruppe (31G) zu einer distalen Endposition der zweiten Elektrodengruppe (32G) erstreckt,
- der dritte Schaftabschnitt (103) ein Schaftabschnitt ist, der sich von der distalen Endposition der zweiten Elektrodengruppe (32G) zu einer proximalen Endposition der zweiten Elektrodengruppe (32G) erstreckt,
- der vierte Schaftabschnitt (104) ein Schaftabschnitt ist, der sich von der proximalen Endposition der zweiten Elektrodengruppe (32G) zu einer proximalen Endposition des distalen Endseitenrohrs (11) erstreckt,
- und der fünfte Schaftabschnitt (105) ein Schaftabschnitt ist, der sich entlang des proximalen Endseitenrohrs (12) erstreckt.

2. Intrakardialer Defibrillationskatheter (100) nach Anspruch 1, wobei die Biegelast L1 des ersten Schaftabschnitts (101) 120 gf oder weniger beträgt und wobei die Biegelast L4 des vierten Schaftabschnitts (104) 350 gf oder mehr beträgt (1 gf = 0,00980665 N).

3. Intrakardialer Defibrillationskatheter (100) nach Anspruch 1 oder 2, wobei, wenn eine Oberflächenhärte des distalen Endseitenrohrs (11), das den ersten Schaftabschnitt (101) bildet, H1 ist und eine Oberflächenhärte des distalen Endseitenrohrs, das den zweiten Schaftabschnitt (102) bildet, H2 ist, ein Unterschied zwischen H2 und H1 16D bis 35D beträgt.

4. Intrakardialer Defibrillationskatheter (100) nach Anspruch 3, wobei, wenn eine Oberflächenhärte des distalen Endseitenrohrs, das den dritten Schaftabschnitt (103) bildet, H3 ist und eine Oberflächenhärte des distalen Endseitenrohrs, das den vierten Schaftabschnitt (104) bildet, H4 ist, ein Unterschied zwischen H4 und H3 2D bis 16D beträgt.

5. Intrakardialer Defibrillationskatheter (100) nach einem der Ansprüche 1 bis 4, wobei, wenn der intrakardiale Defibrillationskatheter (100) in der Herzhöhle angeordnet ist, die erste Elektrodengruppe (31G) in einem Koronarsinus positioniert ist und die zweite Elektrodengruppe (32G) in einem rechten Vorhof positioniert ist.

## Revendications

1. Cathéter de défibrillation intracardiaque (100) doté d'un arbre et comprenant un élément de tube isolant (10)
qui est constitué d'un tube latéral d'extrémité distale (11) et d'un tube latéral d'extrémité proximale (12), d'une poignée (20) qui est reliée à une extrémité proximale de l'élément de tube (10), d'un premier groupe d'électrodes (31G) qui est constitué d'une pluralité d'électrodes en forme d'anneau (31) montées au niveau du tube latéral d'extrémité distale (11) de l'élément de tube (10), et un second groupe d'électrodes (32G) constitué d'une pluralité d'électrodes en forme d'anneau (32) espacées vers un côté d'extrémité proximale par rapport au premier groupe d'électrodes (31G) et montées au niveau du tube latéral d'extrémité distale (11),
le cathéter (100) étant configuré pour effectuer une défibrillation dans une cavité cardiaque en appliquant des tensions ayant des polarités différentes au premier groupe d'électrodes (31G) et au second groupe d'électrodes (32G), dans lequel, en ce qui concerne chacune d'une première partie de l'arbre (101), d'une deuxième partie de l'arbre (102), d'une troisième partie de l'arbre (103), d'une quatrième partie de l'arbre (104) et d'une cinquième partie de l'arbre (105), lorsqu'un essai de flexion en trois points est effectué avec une distance entre les fulcra de 70 mm et une quantité de flexion résultant de l'application d'une charge de flexion à un point intermédiaire de la distance de 10 mm, les charges de flexion L1 à L5 mesurées pour chacune des première à cinquième parties de l'arbre (101, 102, 103, 104, 105) ont une relation de L1 < L2 < L3 < L4 < L5, dans lequel
- la première partie de l'arbre (101) est une partie de l'arbre qui s'étend d'une extrémité distale d'un arbre de cathéter du cathéter de défibrillation intracardiaque (100) à une position d'extrémité proximale du premier groupe d'électrodes (31G),
- la deuxième partie de l'arbre (102) est une partie de l'arbre qui s'étend de la position d'extrémité proximale du premier groupe d'électrodes (31G) à une position d'extrémité distale du second groupe d'électrodes (32G),
- la troisième partie de l'arbre (103) est une partie de l'arbre qui s'étend de la position d'extrémité distale du second groupe d'électrodes (32G) à une position d'extrémité proximale du second groupe d'électrodes (32G),
- la quatrième partie de l'arbre (104) est une partie de l'arbre qui s'étend de la position d'extrémité proximale du second groupe d'électrodes (32G) à une position d'extrémité proximale du tube latéral d'extrémité distale (11),
- et la cinquième partie de l'arbre (105) est une partie de l'arbre qui s'étend le long du tube latéral d'extrémité proximale (12).

2. Cathéter de défibrillation intracardiaque (100) selon la revendication 1, dans lequel la charge de flexion L1 de la première partie de l'arbre (101) est inférieure ou égale à 120 gf, et dans lequel la charge de flexion L4 de la quatrième partie de l'arbre (104) est supérieure ou égale à 350 gf (1 gf = 0,00980665 N).

3. Cathéter de défibrillation intracardiaque (100) selon la revendication 1 ou la revendication 2, dans lequel, lorsqu'une dureté de surface du tube latéral d'extrémité distale (11) qui constitue la première partie de l'arbre (101) est H1 et qu'une dureté de surface du tube latéral d'extrémité distale qui constitue la deuxième partie de l'arbre (102) est H2, une différence entre H2 et H1 est de 16D à 35D.

4. Cathéter de défibrillation intracardiaque (100) selon la revendication 3, dans lequel, lorsqu'une dureté de surface du tube latéral d'extrémité distale qui constitue la troisième partie de l'arbre (103) est H3 et qu'une dureté de surface du tube latéral d'extrémité distale qui constitue la quatrième partie de l'arbre (104) est H4, une différence entre H4 et H3 est de 2D à 16D.

5. Cathéter de défibrillation intracardiaque (100) selon l'une quelconque des revendications 1 à 4, dans lequel, lorsque le cathéter de défibrillation intracardiaque (100) est disposé dans la cavité cardiaque, le premier groupe d'électrodes (31G) est positionné dans un sinus coronaire et le second groupe d'électrodes (32G) est positionné dans une oreillette droite.
